(19) Europäisches Patentamt
European Patent Office
Office européen des brevets

(11) **EP 1 809 232 B1**

(12) **EUROPEAN PATENT SPECIFICATION**

(45) Date of publication and mention of the grant of the patent:
**08.08.2012 Bulletin 2012/32**

(21) Application number: **05792323.7**

(22) Date of filing: **06.10.2005**

(51) Int Cl.:
***A61Q 5/02*** *(2006.01)* ***A61K 8/89*** *(2006.01)*
***A61Q 5/12*** *(2006.01)* ***A61K 8/891*** *(2006.01)*

(86) International application number:
**PCT/EP2005/010863**

(87) International publication number:
**WO 2006/045418 (04.05.2006 Gazette 2006/18)**

(54) **HAIR CARE COMPOSITIONS**

HAARPFLEGEZUSAMMENSETZUNGEN

COMPOSITIONS POUR LE SOIN DES CHEVEUX

(84) Designated Contracting States:
**AT BE BG CH CY CZ DE DK EE ES FI FR GB GR HU IE IS IT LI LT LU LV MC NL PL PT RO SE SI SK TR**

(30) Priority: **20.10.2004 EP 04256459**

(43) Date of publication of application:
**25.07.2007 Bulletin 2007/30**

(73) Proprietors:
- **Unilever PLC**
**London**
**EC4Y 0DY (GB)**
Designated Contracting States:
**CY GB IE**
- **Unilever N.V.**
**3013 AL Rotterdam (NL)**
Designated Contracting States:
**AT BE BG CH CZ DE DK EE ES FI FR GR HU IS IT LI LT LU LV MC NL PL PT RO SE SI SK TR**

(72) Inventors:
- **AVERY, Andrew Richard,**
**Unilever R&D Port Sunlight**
**Wirral,**
**Merseyside CH63 3JW (GB)**
- **KHOSHDEL, Ezat,**
**Unilever R & D Port Sunlight**
**Wirral,**
**Merseyside CH63 3JW (GB)**

(74) Representative: **Newbould, Frazer Anthony et al**
**Unilever PLC**
**Unilever Patent Group**
**Colworth House**
**Sharnbrook**
**Bedford, Bedfordshire MK44 1LQ (GB)**

(56) References cited:
**EP-A- 0 445 982**
**EP-A- 0 963 751**
**US-A1- 2002 098 214**
**US-A1- 2002 168 332**
**US-A1- 2003 055 194**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 809 232 B1

## Description

FIELD OF THE INVENTION

This invention relates to hair care compositions', in particular rinse-off hair care compositions such as conditioners and shampoos, which incorporate branched organosiloxane polymers.

BACKGROUND AND PRIOR ART

Rinse-off hair care compositions typically comprise one or more conditioning agents to provide sensory benefits to the hair.

A problem associated with such rinse-off hair care compositions is that for many consumers they do not provide a sufficient level of sensory benefits such as wet and dry combability, smoothness, softness and shine.

An approach to this problem has been to incorporate organosiloxane polymers as conditioning agents. Organosiloxane polymers can have advantages over organic oils for hair conditioning.

In spite of these advantages, organosiloxane polymers tend to be incorporated into products at relatively low levels. High levels of organosiloxane polymers in products, as well as being costly, can lead to an undesirable build-up on the hair, leading to a greasy or dirty feel.

There is a need for organosiloxane polymers which can provide higher levels of conditioning than current materials; even when present at relatively low levels in a hair care composition.

The present inventors have found that certain branched organosiloxane polymers can improve hair conditioning significantly when they are incorporated into rinse-off hair care compositions such as shampoos and conditioners.

Branched organosiloxane polymers have been described for example in US2003/0055194, which mentions their use as antimisting additives for reducing the formation of aerosols during manufacture of crosslinkable silicone coating compositions for paper, wood, cork and polymer films.

SUMMARY OF THE INVENTION

The present invention provides a hair care composition comprising an organosiloxane polymer having multiple branching points, in which the branching points are linked by organosiloxane segments having a chain length of at least 100 organosiloxy units.

DETAILED DESCRIPTION

Branched Organosiloxane Polymer

The branched organosiloxane polymer for use in the hair care compositions of the invention will generally have a dendritic, such as a hyperbranched, molecular structure. Preferably the viscosity of the branched organosiloxane polymer is at least 8,000 $mm^2sec^{-1}$ at 25 °C, more preferably at least 10,000 $mm^2sec^{-1}$.

Suitable branched organosiloxane polymers for use in the hair care compositions of the invention may be characterised by the general formula (I):

$$Y[-C_nH_{2n}-(R_2SiO)_m-R_2Si-G]_x \qquad (I)$$

where

- Y is a tri- or tetravalent hydrocarbon radical,
- R can be identical or different and is a monovalent hydrocarbon radical having from 1 to 18 carbon atoms per radical,
- G is a monovalent radical of the formula $-C_fH_{2f-2k}-Z$ or a divalent radical of the formula $-C_nH_{2n}-$, the second bond being to a further radical Y,
- Z is a monovalent hydrocarbon radical which is free from terminal aliphatic carbon-carbon multiple bonds, which is inert toward.SiH groups in hydrosilylation reactions,
- x is 3 or 4,
- f is 2,
- k is 0 or 1,

n is an integer from 2 to 4, preferably 2,
m is an integer of at least 100 and

with the proviso that the branched organosiloxane polymers contain on average at least one group Z.

Branched organosiloxane polymers of the above type may suitably be made by a process involving hydrosilylation, as described for example in US2003/0055194.

A typical process comprises in a first step reacting compounds (1) containing three or four aliphatic.double bonds, of the formula

$$Y(CR^1{=}CH_2)_x$$

where Y and x are as defined' above and $R^1$ is a hydrogen atom or an alkyl radical having from 1 to 10 carbon atoms with organopolysiloxanes (2) of the general formula

$$H(R_2SiO)_m{-}R_2SiH$$

where R and m are as defined above, in the presence of catalysts (3) which promote the addition of Si-bonded hydrogen onto aliphatic multiple bond, known as hydrosilylation catalysts, and in a second step reacting the resulting branched intermediates (5) containing Si-bonded hydrogen atoms with organic compounds (4) of the formula

$$C_2H_{3-2k}{-}Z$$

selected from the group consisting of
if k=0 :

$$H_2C{=}CR^3{-}Z \quad (4a)$$

and
if k=1 :

$$R^4C{\equiv}C{-}Z \quad (4b)$$

in which $R^3$ and $R^4$ have the definition of $R^1$ and f, k and Z are as defined above in the presence of catalysts (3) which promote the addition of Si-bonded hydrogen onto aliphatic multiple bond.

The branched organosiloxane.polymers as described in the above general formula comprise chainlike siloxane blocks whose ends are connected to the structural elements Y and/or Z by way in each case of a $C_fH_{2f}$ or $C_fH_{2f-2}$ bridge . The greater the extent to which siloxane blocks are connected on both sides to elements Y, the greater the degree of branching in the polymer. In general, the construction of the branched organosiloxane polymers is such that siloxane blocks and organic blocks alternate with one another, with the branching structures and the ends being composed of organic blocks.

The radical R is preferably a monovalent hydrocarbon radical having from 1 to 6 carbon atoms, the methyl radical being particularly preferred.

Examples of compounds (1) with which the branched organosiloxane polymers can be prepared are 1,2,4-trivinylcyclohexane, 1,3,5-trivinylcyclohexane, 3,5-dimethyl-4-vinyl-1,6- heptadiene, 1,2,3,4-tetravinylcyclobutane, methyltrivinylsilane, tetravinylsilane, and 1,1,2,2-tetraallyloxyethane. A preferred compound (1) is 1,2,4-trivinylcyclohexane.

The radical Z is preferably a monovalent hydrocarbon radical having from 6 to 16 carbon atoms, preferably hexyl.

Preferably m ranges from 100 to 300, more preferably from 200 to' 300.

A specific example of a preferred branched organosiloxane polymer suitable for use in the hair care compositions

of the invention may be characterised by the formula (II):

$$C_6H_9[-C_2H_4-(Me_2SiO)_m-Me_2Si-G]_3 \qquad (II)$$

in which m ranges from 200 to 250, preferably about 220 to 240, G is a mixture of $-C_2H_4-$ and $-C_2H_4-C_6H_{13}$ groups and the group "$C_6H_9$" corresponds to a trivalent "1.2.4-cyclohexane triyl"-radical. The molar ratio of $-C_2H_4-$ to $-C_2H_4-C_6H_{13}$ groups in the mixture G is typically about 2:1.

For ease of processing, the branched organosiloxane polymer can be blended with a diluent. Examples of suitable diluents include hydrocarbon solvents and silicone solvents. Silicone solvents are preferred. Examples of suitable silicone solvents are cyclic silicones and dimethicone fluids. Preferred are dimethicone fluids having a degree of polymerisation ranging from 10 to 20 and a viscosity of 5 to 15 $mm^2sec^{-1}$ (centistokes) at 25°C. Commercially available examples of cyclic silicones and dimethicone fluids are DC 244, 245, 344, 345 and 200 fluids from Dow Corning, with a preferred example being AK10 dimethicone fluid from Wacker-Chemie GmbH.

The blend of branched organosiloxane polymer and diluent can be incorporated into the hair care composition of the invention by mixing, mechanical dispersion, or preferably by first forming an emulsion of the blend using a suitable emulsifier, and then mixing the emulsion with the remaining ingredients of the composition. Suitable emulsifiers used to form the emulsion include anionic, cationic and nonionic surfactants.

The total amount of branched organosiloxane polymer in hair care compositions of the invention generally ranges from 0.1 to 5%, preferably from 0.15 to 3%, more preferably from 0.2 to 2% by total weight branched organosiloxane polymer based on the total weight of the composition.

Product Form

Compositions of the invention are typically "rinse-off" compositions to be applied to the hair and then rinsed away.

A particularly preferred product form for compositions in accordance with the invention is a conditioner for the treatment of hair (typically after shampooing) and subsequent rinsing.

Conditioner Compositions

such conditioner compositions comprise one or more conditioning surfactants which are cosmetically acceptable and suitable for topical application to the hair.

Suitable conditioning surfactants are selected from cationic surfactants, used singly or in admixture. Preferably, the cationic surfactants have the formula $N^+R^1R^2R^3R^4$ wherein $R^1$, $R^2$, $R^3$ and $R^4$ are independently ($C_1$ to $C_{30}$) alkyl or benzyl. Preferably, one, two or three of $R^1$, $R^2$, $R^3$ and $R^4$ are independently ($C_4$ to $C_{30}$) alkyl and the other $R^1$, $R^2$, $R^3$ and $R^4$ group or groups are ($C_1$-$C_6$) alkyl or benzyl. More preferably, one' or two of $R^1$, $R^2$, $R^3$ and $R^4$ are independently ($C_6$ to $C_{30}$) alkyl and the other $R^1$, $R^2$, $R^3$ and $R^4$ groups are ($C_1$-$C_6$) alkyl or benzyl groups. Optionally, the alkyl groups may comprise one or more ester (-OCO- or -COO-) and/or ether (-O-) linkages within the alkyl chain. Alkyl groups may optionally be substituted with one or more hydroxyl groups. Alkyl groups may be straight chain or branched and, for alkyl groups having 3 or more carbon atoms, cyclic. The alkyl groups may be saturated or may contain one or more carbon-carbon double bonds (e.g., oleyl). Alkyl groups are optionally ethoxylated on the alkyl chain with one or more ethyleneoxy groups.

Suitable cationic surfactants for use in conditioner compositions according to the invention include cetyltrimethylammonium chloride, behenyltrimethylammonium chloride, cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, dihydrogenated tallow dimethyl ammonium chloride (e.g., Arquad 2HT/75 from Akzo Nobel), cocotrimethylammonium chloride, PEG-2-oleammonium chloride and the corresponding hydroxides thereof. Further suitable cationic surfactants include those materials having the CTFA designations Quaternium-5, Quaternium-31 and Quaternium-18. Mixtures of any of the foregoing materials may also be suitable. A particularly useful cationic surfactant for use in conditioners according to the invention is cetyltrimethylammonium chloride, available commercially, for example as GENAMIN CTAC, ex Hoechst Celanese. Another particularly useful cationic surfactant for use in conditioners according to the invention is behenyltrimethylammonium chloride, available commercially, for example as GENAMIN KDMP, ex Clariant.

Another example of a class of suitable cationic surfactants for use in the invention, either alone or together with one or more other cationic surfactants, is a combination of (i) and (ii) below:

(i) an amidoamine corresponding to the general formula (I):

in which $R^1$ is a hydrocarbyl chain having 10 or more carbon atoms,
$R^2$ and $R^3$ are independently selected from hydrocarbyl chains of from 1 to 10 carbon atoms, and
m is an integer from 1 to about 10; and

(ii) an acid.

As used herein, the term hydrocarbyl chain means an alkyl or alkenyl chain.
Preferred amidoamine compounds are those corresponding to formula (I) in which

$R^1$ is a hydrocarbyl residue having from about 11 to about 24 carbon atoms,
$R^2$ and $R^3$ are each independently hydrocarbyl residues, preferably alkyl groups, having from 1 to about 4 carbon atoms, and
m is an integer from 1 to about 4.

Preferably, $R^2$ and $R^3$ are methyl or ethyl groups.
Preferably, m is 2 or 3, i.e. an ethylene or propylene group.
Preferred amidoamines useful herein include stearamidopropyldimethylamine, stearamidopropyldiethylamine, stearamidoethyldiethylamine, stearamidoethyldimethylamine, palmitamidopropyldimethylamine, palmitamidopropyldiethylamine, palmitamidoethyldiethylamine, palmitamidoethyldimethylamine, behenamidopropyldimethylamine, behenamidopropyldiethylmine, behenamidoethyldiethylamine, behenamidoethyldimethylamine, arachidamidopropyldimethylamine, arachidamidopropyldiethylamine, arachid-amidoethyldiethylamine, arachidamidoethyldimethylamine, and mixtures thereof.
Particularly preferred amidoamines useful herein are stearamidopropyldimethylamine, stearamidoethyldiethylamine, and mixtures thereof.
Commercially available amidoamines useful herein include: stearamidopropyldimethylamine with tradenames LEXAMINE S-13 available from Inolex (Philadelphia Pennsylvania, USA) and AMIDOAMINE MSP available from Nikko (Tokyo, Japan), stearamidoethyldiethylamine with a tradename AMIDOAMINE S available from Nikko, behenamidopropyldimethylamine with a tradename INCROMINE BB available from Croda (North Humberside, England), and various amidoamines with tradenames SCHERCODINE series available from Scher (Clifton New Jersey, USA).
Acid (ii) may be any organic or mineral acid which is capable of protonating the amidoamine in the hair treatment composition. Suitable acids useful herein include hydrochloric acid, acetic acid, tartaric acid, fumaric acid, lactic acid, malic acid,' succinic acid, and mixtures thereof. Preferably, the acid is selected from the group consisting of acetic acid, tartaric acid, hydrochloric acid, fumaric acid, and mixtures thereof.
The primary role of the acid is to protonate the amidoamine in the hair treatment composition thus forming a tertiary amine salt (TAS) in situ in the hair treatment composition. The TAS in effect is a non-permanent quaternary ammonium or pseudo-quaternary ammonium cationic surfactant.
Suitably, the acid is included in a sufficient amount to protonate all the amidoamine present, i.e. at a level which is at least equimolar to the amount of amidoamine present in the composition.
In conditioners of the invention, the level of cationic surfactant will generally range from 0.01 to 10%, more preferably 0.05 to 7.5%, most preferably 0.1 to 5% by weight of the composition.
Conditioners of the invention will typically also incorporate a fatty alcohol. The combined use of fatty alcohols and cationic surfactants in conditioning compositions is believed to be especially advantageous, because this leads to the formation of a lamellar phase, in which the cationic surfactant is dispersed.
Representative fatty alcohols comprise from 8 to 22 carbon atoms, more preferably 16 to 22. Fatty alcohols are typically compounds containing straight chain alkyl groups. Examples of suitable fatty alcohols include cetyl alcohol, stearyl alcohol and mixtures thereof. The use of these materials is also advantageous in that they contribute to the overall conditioning properties of compositions of the invention.
The level of fatty alcohol in conditioners of the invention will generally range from 0.01 to 10%, preferably from 0.1 to 8%, more preferably from 0.2 to 7%, most preferably from 0.3 to 6% by weight of the composition. The weight

ratio of cationic surfactant to fatty alcohol is suitably from 1:1 to 1:10, preferably from 1:1.5 to 1:8, optimally from 1:2 to 1:5. If the weight ratio of cationic surfactant to fatty alcohol is too high, this can lead to eye irritancy from the composition. If it is too low, it can make the hair feel squeaky for some consumers.

Another preferred product form is a shampoo composition.

Shampoo Composition

Shampoo compositions of the invention are generally aqueous, i.e. they have water or an aqueous solution or a lyotropic liquid crystalline phase as their major component. Suitably, the composition will comprise from 50 to 98%, preferably from 60 to 90% water by weight based on the total weight of the composition.

Anionic Cleansing Surfactant

Shampoo compositions according to the invention comprise one or more anionic cleansing surfactants which are cosmetically acceptable and suitable for topical application to the hair.

Examples of suitable anionic cleansing surfactants are the alkyl sulphates, alkyl ether sulphates, alkaryl sulphonates, alkanoyl isethionates, alkyl succinates, alkyl sulphosuccinates, alkyl ether sulphosuccinates, N-alkyl sarcosinates, alkyl phosphates, alkyl ether phosphates, and alkyl ether carboxylic acids and salts thereof, especially their sodium, magnesium, ammonium and mono-, di- and triethanolamine salts. The alkyl and acyl groups generally contain from 8 to 18, preferably from 10 to 16 carbon atoms and may be unsaturated. The alkyl ether sulphates, alkyl ether sulphosuccinates, alkyl ether phosphates and alkyl ether carboxylic acids and salts thereof may contain from 1 to 20 ethylene oxide or propylene oxide units per molecule.

Typical anionic cleansing surfactants for use in shampoo compositions of the invention include sodium oleyl succinate, ammonium lauryl sulphosuccinate, sodium lauryl sulphate, sodium lauryl ether sulphate, sodium lauryl ether sulphosuccinate, ammonium lauryl sulphate, ammonium lauryl ether sulphate, sodium dodecylbenzene sulphonate, triethanolamine dodecylbenzene sulphonate, sodium cocoyl isethionate, sodium lauryl isethionate, lauryl ether carboxylic acid and sodium N-lauryl sarcosinate.

Preferred anionic cleansing surfactants are sodium lauryl sulphate, sodium lauryl ether sulphate (n)EO, (where n is from 1 to 3), sodium lauryl ether sulphosuccinate(n)EO, (where n is from 1 to 3), ammonium lauryl sulphate, ammonium lauryl ether sulphate(n)EO, (where n is from 1 to 3), sodium cocoyl isethionate and lauryl ether carboxylic acid (n) EO (where n is from 10 to 20).

Mixtures of any of the foregoing anionic cleansing surfactants may also be suitable.

The total amount of anionic cleansing surfactant in shampoo compositions of the invention generally ranges from 0.5 to 45%, preferably from 1.5 to 35%, more preferably from 5 to 20% by total weight anionic cleansing surfactant based on the total weight of the composition.

Further Ingredients

Optionally, a shampoo composition of the invention may contain further ingredients as described below to enhance performance and/or consumer acceptability.

Co-surfactant

The composition can include co-surfactants, to help impart aesthetic, physical or cleansing properties to the composition.

An example of a co-surfactant is a nonionic surfactant, which can be included in an amount ranging from 0.5 to 8%, preferably from 2 to 5% by weight based on the total weight of the composition.

For example, representative nonionic surfactants that can be included in shampoo compositions of the invention include condensation products of aliphatic ($C_8$ - $C_{18}$) primary or secondary linear or branched chain alcohols or phenols with alkylene oxides, usually ethylene oxide and generally having from 6 to 30 ethylene oxide groups.

Other representative nonionic surfactants include mono- or di-alkyl alkanolamides. Examples include coco mono- or di-ethanolamide and coco mono-isopropanolamide.

Further nonionic surfactants which can be included in shampoo compositions of the invention are the alkyl polyglycosides (APGs). Typically, the APG is one which comprises an alkyl group connected (optionally via a bridging group) to a block of one or more glycosyl groups. Preferred APGs are defined by the following formula:

$$RO - (G)_n$$

therein R is a branched or straight chain alkyl group which may be saturated or unsaturated and G is a saccharide group.

R may represent a mean alkyl chain length of from about $C_5$ to about $C_{20}$. Preferably R represents a mean alkyl chain length of from about $C_8$ to about $C_{12}$. Most preferably the value of R lies between about 9.5 and about 10.5. G may be selected from $C_5$ or $C_6$ monosaccharide residues, and is preferably a glucoside. G may be selected from the group comprising glucose, xylose, lactose, fructose, mannose and derivatives thereof. Preferably G is glucose.

The degree of polymerisation, n, may have a value of from about 1 to about 10 or more. Preferably, the value of n lies from about 1.1 to abut 2. Most preferably the value of n lies from about 1.3 to about 1.5.

Suitable alkyl polyglycosides for use in the invention are commercially available and include for example those materials identified as: Oramix NS10 ex Seppic; Plantaren 1200 and Plantaren 2000 ex Henkel.

Other sugar-derived nonionic surfactants which can be included in compositions of the invention include the $C_{10}$-$C_{18}$ N-alkyl ($C_1$-$C_6$) polyhydroxy fatty acid amides, such as the $C_{12}$-$C_{18}$ N-methyl glucamides, as described for example in WO 92 06154 and US 5 194 639, and the N-alkoxy polyhydroxy fatty acid amides, such as $C_{10}$-$C_{18}$ N-(3-methoxypropyl) glucamide.

A preferred example of a co-surfactant is an amphoteric or zwitterionic surfactant, which can be included in an amount ranging from 0.5 to about 8%, preferably from 1 to 4% by weight based on the total weight of the composition.

Examples of amphoteric or zwitterionic surfactants include alkyl amine oxides, alkyl betaines, alkyl amidopropyl betaines, alkyl sulphobetaines (sultaines), alkyl glycinates, alkyl carboxyglycinates, alkyl amphoacetates, alkyl amphopropionates, alkylamphoglycinates, alkyl amidopropyl hydroxysultaines, acyl taurates and acyl glutamates, wherein the alkyl and acyl groups have from 8 to 19 carbon atoms. Typical amphoteric and zwitterionic surfactants for use in shampoos of the invention include lauryl amine oxide, cocodimethyl sulphopropyl betaine, lauryl betaine, cocamidopropyl betaine and sodium cocoamphoacetate.

A particularly preferred amphoteric or zwitterionic surfactant is cocamidopropyl betaine.

Mixtures of any of the foregoing amphoteric or zwitterionic surfactants may also be suitable. Preferred mixtures are those of cocamidopropyl betaine with further amphoteric or zwitterionic surfactants as described above. A preferred further amphoteric or zwitterionic surfactant is sodium cocoamphoacetate.

The total amount of surfactant (including any co-surfactant, and/or any emulsifier) in a -shampoo composition of the invention is generally from 1 to 50%, preferably from 2 to 40%, more preferably from 10 to 25% by total weight surfactant based on the total weight of the composition.

Cationic Polymers

Cationic polymers are preferred ingredients in a shampoo composition of the invention for enhancing conditioning performance.

Suitable cationic polymers may be homopolymers which are cationically substituted or may be formed from two or more types of monomers. The weight average ($M_w$) molecular weight-of the polymers will generally be between 100 000 and 2 million daltons. The polymers will have cationic nitrogen containing groups such as quaternary ammonium or protonated amino groups, or a mixture thereof. If the molecular weight of the polymer is too low, then the conditioning effect is poor. If too high, then there may be problems of high extensional viscosity leading to stringiness of the composition when it is poured.

The cationic nitrogen-containing group will generally be present as a substituent on a fraction of the total monomer units of the cationic polymer. Thus when the polymer is not a homopolymer it can contain spacer non-cationic monomer units. Such polymers are described in the CTFA Cosmetic Ingredient Directory, 3rd edition. The ratio of the cationic to non-cationic monomer units is selected to give polymers having a cationic charge density in the required range, which is generally from 0.2 to 3.0 meq/gm. The cationic charge density of the polymer is suitably determined via the Kjeldahl method as described in the US Pharmacopoeia under chemical tests for nitrogen determination.

Suitable cationic polymers include, for example, copolymers of vinyl monomers having cationic amine or quaternary ammonium functionalities with water soluble spacer monomers such as (meth)acrylamide, alkyl and dialkyl (meth)acrylamides, alkyl (meth) acrylate, vinyl caprolactone and vinyl pyrrolidine. The alkyl and dialkyl substituted monomers preferably have C1-C7 alkyl groups, more preferably C1-3 alkyl groups. Other suitable spacers include vinyl esters, vinyl alcohol, maleic anhydride, propylene glycol and ethylene glycol.

The cationic amines can be primary, secondary or tertiary amines, depending upon the particular species and the pH of the composition. In general secondary and tertiary amine, especially tertiary, are preferred.

Amine substituted vinyl monomers and amines can be polymerised in the amine form and then converted to ammonium by quaternization.

The cationic polymers can comprise mixtures of monomer units derived from amine- and/or quaternary ammonium-substituted monomer and/or compatible spacer monomers.

Suitable cationic polymers include, for example:

- cationic diallyl quaternary ammonium-containing polymers including, for example, dimethyldiallylammonium chloride homopolymer and copolymers of acrylamide and dimethyldiallylammonium chloride, referred to in the industry (CTFA) as Polyquaternium 6 and Polyquaternium 7, respectively;

- mineral acid salts of amino-alkyl esters of homo-and co-polymers of unsaturated carboxylic acids having from 3 to 5 carbon atoms, (as described in U.S. Patent 4, 009, 256) ;

- cationic polyacrylamides(as described in WO95/22311).

Other cationic polymers that can be used include cationic polysaccharide polymers, such as cationic cellulose derivatives, cationic starch derivatives, and cationic guar gum derivatives.

Cationic polysaccharide polymers suitable for use in compositions of the invention include monomers of the formula:

$$A\text{-}O\text{-}[R\text{-}N^{+}(R^{1})\ (R^{2})\ (R^{3})X^{-}],$$

wherein: A is an anhydroglucose residual group, such as a starch or cellulose anhydroglucose residual. R is an alkylene, oxyalkylene,polyoxyalkylene, or hydroxyalkylene group, or combination thereof. $R^1$, $R^2$ and $R^3$ independently represent alkyl, aryl, alkylaryl, arylalkyl, alkoxyalkyl, or alkoxyaryl groups, each group containing up to about 18 carbon atoms. The total number of carbon atoms for each cationic moiety (i.e., the sum of carbon atoms in $R^1$, $R^2$ and $R^3$) is preferably about 20 or less, and X is an anionic counterion.

Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from the Amerchol Corporation, for instance under the tradename Polymer LM-200.

Other suitable cationic polysaccharide polymers include quaternary nitrogen-containing cellulose ethers (e.g. as described in U.S. Patent 3,962,418), and copolymers of etherified cellulose and starch (e.g. as described in U.S. Patent 3, 958, 581) .

A particularly suitable type of cationic polysaccharide polymer that can be used is a cationic guar gum derivative, such as guar hydroxypropyltrimethylammonium chloride (commercially available from Rhodia in their JAGUAR trademark series). Examples of such materials are JAGUAR C13S, JAGUAR C14, JAGUAR C15, JAGUAR C17 and JAGUAR C16 Jaguar CHT and JAGUAR C162..

Mixtures of any of the above cationic polymers may be used.

Cationic polymer will generally be present in a shampoo composition of the invention at levels of from 0.01 to 5%, preferably from 0.05 to 1%, more preferably from 0.08 to 0.5% by total weight of cationic polymer based on the total weight of the composition.

Suspending Agent

Preferably an aqueous shampoo composition of the inventions further comprises a suspending agent. Suitable suspending agents are selected from polyacrylic acids, cross-linked polymers of acrylic acid, copolymers of.acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearate, alkanolamides of fatty acids having from 16 to 22 carbon atoms and mixtures thereof. Ethylene glycol distearate and polyethylene glycol 3 distearate are preferred long chain acyl derivatives, since these impart pearlescence to the composition. Polyacrylic acid is available commercially as Carbopol 420, Carbopol 488 or Carbopol 493. Polymers of acrylic acid cross-linked with a polyfunctional agent may also be used; they are available commercially as Carbopol 910, Carbopol 934, Carbopol 941 and Carbopol 980. An example of a suitable copolymer of a carboxylic acid containing monomer and acrylic acid esters is Carbopol 1342. All Carbopol (trademark) materials are available from Goodrich.

Suitable cross-linked polymers of acrylic,acid and acrylate esters are Pemulen TR1 or Pemulen TR2. A suitable heteropolysaccharide gum is xanthan gum, for example that available as Kelzan mu.

Mixtures of any of the above suspending agents may be used. Preferred is a mixture of cross-linked polymer of acrylic acid and crystalline long chain acyl derivative.

Suspending agent will generally be present in a shampoo composition of the invention at levels of from 0.1 to 10%, preferably from 0.5 to 6%, more preferably from 0.9 to 4% by total weight of suspending agent based on the total weight of the composition.

Further Conditioning Agents

Compositions of the invention may comprise further conditioning agents, in addition to the branched organosiloxane polymer as described above, in order to optimise wet and dry conditioning benefits.

Particularly preferred further conditioning agents are additional silicone emulsions such as those formed from silicones such as polydiorganosiloxanes, in particular polydimethylsiloxanes which have the CTFA designation dimethicone, polydimethyl siloxanes having hydroxyl end groups which have the CTFA designation dimethiconol, and amino-functional polydimethyl siloxanes which have the CTFA designation amodimethicone.

The emulsion droplets may typically have a Sauter mean droplet diameter ($D_{3,2}$) in the composition of the invention ranging from 0.01 to 20 micrometer, more preferably from 0.2 tao 10 micrometer.

A suitable method for measuring the Sauter mean droplet diameter ($D_{3,2}$) is by laser light scattering using an instrument such as a Malvern Mastersizer.

Suitable silicone emulsions for use in compositions of the invention are available from suppliers of silicones such as Dow Corning and GE Silicones. The use of such pre-formed silicone emulsions is preferred for ease of processing and control of silicone particle size. Such pre-formed silicone emulsions will typically additionally comprise a suitable emulsifier such as an anionic or nonionic emulsifier, or mixture thereof, and may be prepared by a chemical emulsification process such as emulsion polymerisation, or by mechanical emulsification using a high shear mixer. Pre-formed silicone emulsions having a Sauter mean droplet diameter ($D_{3,2}$) of less than 0.15. micrometers are gene'rally termed microemulsions.

Examples of suitable pre-formed silicone emulsions include emulsions DC2-1766, DC2-1784, DC-1785, DC-1786, DC-1788 and microemulsions DC2-1865 and DC2-1870, all available from Dow Corning. These are all emulsions/microemulsions of dimethiconol. Also suitable are amodimethicone emulsions such as DC2-8177 and DC939 (from Dow Corning) and SME253 (from GE Silicones).

Also suitable are silicone emulsions in which certain types of surface active block copolymers of a high molecular weight have been blended with the silicone emulsion droplets, as described for example in WO03/094874. In such materials, the silicone emulsion droplets are preferably formed from polydiorganosiloxanes such as those described above. One preferred form of the surface active bloc copolymer.is according to the following formula:

$$HO(CH_2CH_2O)_x(\underset{\displaystyle CH_3}{\underset{|}{C}}HCH_2O)_y(CH_2CH_2O)_x H$$

wherein the mean value of x is 4 or more and the mean value of y is 25 or more.

Another preferred form of the surface active block copolymer is according to the following formula:

$$(HO(CH_2CH_2O)_a(\underset{\displaystyle CH_3}{\underset{|}{C}}HCH_2O)_b)_2-N-CH_2-CH_2-N((OCH_2\underset{\displaystyle CH_3}{\underset{|}{C}}H)_b(OCHCH_2)_aOH)_2$$

wherein the mean value of a is 2 or more and the mean value of b is 6 or more.

Mixtures of any of the above described silicone emulsions may also be used.

The above described silicone emulsions will generally be present in a composition of the invention at levels of from 0.05 to 10%, preferably 0.05 to 5%, more preferably from 0.5 to 2% by total weight of silicone based on the total weight of the composition.

Other Optional Ingredients

A composition of the invention may contain other ingredients for enhancing performance and/or consumer acceptability. Such ingredients include fragrance, dyes and pigments, pH adjusting agents, pearlescers or opacifiers, viscosity modifiers, preservatives, and natural hair nutrients such as botanicals, fruit extracts, sugar derivatives and amino acids.

Mode of Use

The compositions of the invention are primarily intended for topical application to the hair and/or scalp of a human subject in rinse-off compositions, in order to improve hair fibre surface properties such as smoothness, softness, manageability, cuticle integrity, and shine.

The compositions provided by the invention are preferably conditioner compositions for the treatment of hair (typically after shampooing) and subsequent rinsing.

Alternatively the compositions provided by the invention may be aqueous shampoo compositions, used by massaging them into the hair followed by rinsing with clean water prior to drying the hair. Optionally, a separate conditioning formulation may be applied after rinsing and before drying, but this may not be necessary as an aqueous shampoo composition of this invention is intended to provide both cleansing and conditioning to the hair.

The invention is further illustrated with.reference to the following, non-limiting examples, in which all percentages are by weight based on total weight unless otherwise specified. Examples according to the invention are indicated by a number. Comparative examples (not according to the invention) are indicated by a letter.

EXAMPLES

Hair conditioner formulations were prepared having ingredients as shown in the following Table:

| Ingredient | Ex. 1 | Comp. Ex.A | Comp. Ex.B | Comp. Ex.C |
|---|---|---|---|---|
| Cetrimonium chloride (29% a.i.) | 5.8 | 5.8 | 5.8 | 5.8 |
| Di (hydrogenated tallow) dimethylammonium chloride (75%) /2-propanol (14%) | 1 | 1 | 1 | 1 |
| Cetearyl alcohol | 5 | 5 | 5 | 5 |
| Silicone emulsion(1) | 6 | - | - | - |
| ilicone emulsion(2) | - | 6 | - | - |
| Silicone emulsion(3) | - | - | 6 | |
| Silicone emulsion(4) | - | - | - | 5 |
| Methyl paraben | 0.2 | 0.2 | 0.2 | 0.2 |
| Water | to 100 | to 100 | to 100 | to 100 |

(1) Emulsion (50wt% a.i. with isolaureth-6 and water) of a 50:50 w/w'mix of branched organosiloxane polymer of formula (II) above in which m is 220 to 240, (from Wacker-Chemie GmbH), and 10 $mm^2sec^{-1}$ (centistokes) dimethicone diluent (AK10 dimethicone fluid from Wacker-Chemie GmbH).
(2) Emulsion (50% a.i. with.isolaureth-6 and water) of a. branched organosiloxane polymer of formula (II) above in which m is 55, (from Wacker-Chemie.GmbH).
(3) Emulsion (50% a.i. with isolaureth-6 and water) of a branched organosiloxane polymer of formula (II) above in which m is 15, (from Wacker-Chemie GmbH).
(4) Emulsion (60% a.i.) of dimethiconol and TEA-dodecylbenzenesulphonate. Average particle size of the emulsion is between 0.5 and 1.0 micron. Viscosity of the dimethiconol is at least 1 million $mm^2sec^{-1}$ at 25°C.

In vitro evaluation of friction of dry hair following treatment with example conditioners

The conditioners as described above were evaluated in a comparative test according to the following protocol:

European dark brown hair was used in switches of 2.5 g weight and 6 inches length.

Each conditioner was tested on 5 such hair switches.

Switch treatment

Each switch was first rinsed under running tap water for 30 seconds. A basic cleansing shampoo (0.5 ml) was applied to each switch and the hair agitated with gloved fingers for 30 seconds followed by further rinsing for 30 seconds

under running tap water. This process was repeated once more.

Each hair switch, after the procedure above, was then treated with individual test conditioners as follows:

Each switch was first rinsed under running tap water for 10 seconds before the conditioner (0.5 ml) under test was applied and massaged into the hair for 1 minute. The test conditioner was then rinsed under running tap water for 1 minute.

The switches were then dried at 50° C for 30 minutes and left to equilibrate under temperature and humidity controlled conditions overnight. Friction was measured using the texture analyser to obtain the dry friction value.

Friction measurement methodology

All friction measurements were carried out under controlled conditions of 20 degrees and 50 % RH.

Friction was measured using a TA.XT2i Texture Analyser supplied by Stable Micro Systems, Survey, UK. The friction probe was a stainless steel cylinder, which was coated with rubber material. The load on the friction contact was approximately 560 g. When in use, an area of contact between the friction probe.. and the hair of approximately 1.0 $cm^2$ was achieved.

The methodology used to assess the friction properties of hair treated with test conditioners was as follows:

A switch of hair was securely mounted onto the texture analyser, the hair fibres being aligned by combing before being secured in a flat configuration. The friction probe was placed onto the hair and moved along the hair at a speed of 10 $mms^{-1}$ to measure the friction between the probe and the hair.

The friction values reported below are of friction hysteresis in units of g.mm, obtained by integrating the total measured friction force over the total distance travelled by the probe, with and against cuticle. Two measurements were performed per switch and 5 switches per formulation were tested. The values reported are the mean values derived from these ten readings per treatment.

Results

| Test Conditioner | Dry friction test/ mean friction hysteresis (g.mm) | Dry friction test/ standard error |
|---|---|---|
| Comparative Example B | 22161 | 748 |
| Comparative Example A | 33779 | 470 |
| Example 1 | 17844 | 615 |
| Comparative Example C | 23735 | 1060 |

The results show that Example 1 according to the invention delivers superior lubrication to dry hair than any of Comparative Examples A to C.

Further hair conditioner formulations were prepared having ingredients as shown in the following Table:

| Ingredient | Comp. Ex.D | Ex.2 | Ex.3 | Ex.4 | Ex.5 | Ex.6 | Ex.7 | Ex.8 | Ex.9 | Ex.10 | Ex.11 | Ex.12 | Ex.13 | Ex.14 | Ex.15 | Ex.16 |
|---|---|---|---|---|---|---|---|---|---|---|---|---|---|---|---|---|
| Cetrimonium chloride (29% a.i.) | 5.5 | 5.4 | 5.1 | 5.1 | 5.3 | 5.3 | 5.3 | 5.6 | 5.4 | 5.3 | 5.3 | 5.4 | 5.2 | 5.1 | 5.1 | 5.1 |
| Di (hydrogenated tallow) dimethyl ammonium chloride (75%) / 2-propanol (14%) | 1 | 1 | 0.9 | 0.9 | 0.9 | 0.9 | 0.9 | 1 | 1 | 0.9 | 0.9 | 0.9 | 0.9 | 0.9 | 0.9 | 0.9 |
| Cetearyl alcohol | 5.7 | 5.6 | 5.3 | 5.3 | 5.5 | 5.5 | 5.5 | 5.8 | 5.6 | 5.5 | 5.5 | 5.6 | 5.4 | 5.3 | 5.3 | 5.3 |
| Silicone (5) | 0 | 3 | 6 | 6 | 4 | 4 | 4 | 2 | 3 | 4 | 4 | 1.5 | 4.5 | 5.4 | 1.5 | 0.6 |
| Isolaureth-6 | 0 | 0.3 | 0.7 | 0.4 | 0.4 | 0.3 | 0.2 | 0.1 | 0.3 | 0.4 | 0.3 | 0.4 | 0.6 | 0.6 | 0.4 | 0.4 |
| Dimethicone (DC200 fluid, 600,000 $mm^2s^{-1}$ (centistokes), from Dow Corning) | 0 | 0 | 0 | 0 | 0 | 0 | 0 | 0 | 0 | 0 | 0 | 2.25 | 0.75 | 0.3 | 2.25 | 2.7 |
| Silicone emulsion(4) | 5 | 0 | 0 | 0 | 0 | 0 | 0 | 0 | 0 | 0 | 0 | 0 | 0 | 0 | 0 | 0 |
| Methyl paraben | 0.2 | 0.2 | 0.2 | 0.2 | 0.2 | 0.2 | 0.2 | 0.2 | 0.2 | 0.2 | 0.2 | 0.2 | 0.2 | 0.2 | 0.2 | 0.2 |
| Water | to 100 | to 100 | to 100 | to 100 | to 100 | to 100 | to 100 | to 100 | to 100 | to 100 | to 100 | to 100 | to 100 | to 100 | to 100 | to 100 |

(5) A 50:50 w/w mix of branched organosiloxane polymer of formula (II) above in which m is 220 to 240, (from Wacker-Chemie GmbH), and 10 $mm^2sec^{-1}$ (centistokes) dimethicone diluent (AK10 dimethicone fluid from Wacker-Chemie GmbH).

In the formulations of Examples 2 to 16 above, the organosiloxane polymer (5) is emulsified with the isolaureth-6. In the formulations of Examples 12 to 16, the polymer (5) is premixed with the dimethicone prior to emulsification emulsified with the isolaureth-6.

The conditioners were evaluated using the friction measurement methodology as described above. The results were as follows:

| Test Conditioner | Dry friction test/mean friction hysteresis (g.mm) | Dry friction test/standard error |
|---|---|---|
| Comparative Example D | 22258 | 739 |
| Example 2 | 17844 | 615 |
| Example 3 | 14575 | 170 |
| Example 4 | 14524 | 380 |
| Example 5 | 17980 | 302 |
| Example 6 | 16941 | 340 |
| Example 7 | 15621 | 314 |
| Example 8 | 20311 | 587 |
| Example 9 | 15372 | 288 |
| Example 10 | 14784 | 299 |
| Example 11 | 14877 | 287 |
| Example 12 | 12921 | 118 |
| Example 13 | 13520 | 138 |
| Example 14 | 13260 | 219 |
| Example 15 | 12790 | 263 |
| Example 16 | 13738 | 240 |

The results show that, when compared at an identical weight percentage active ingredient in a conditioner formulation, the silicone (5) provides greater lubrication than the control silicone of Comparative Example E (silicone emulsion (4)). Also, silicone(5) can be used at a lower weight percentage active ingredient than the control silicone (reducing the total silicone in the formulation), yet still provides at least the same degree of lubrication to dry hair as shown by the in vitro measurements. The combination of silicone(5) with dimethicone (Examples 12 to 16) delivers improved dry lubrication relative to the same level of either silicone(5) or control silicone.

Further hair conditioner formulations were prepared having ingredients as shown in the following Table:

| Ingredient | Comp. Ex.E | Comp. Ex.F | Ex.17 | Ex.18 |
|---|---|---|---|---|
| Cetrimonium chloride (50% a.i.) | 3.24 | 3.24 | 3.24 | 3.24 |
| Di(hydrogenated tallow) dimethylammonium chloride (75%) / 2-propanol (14%) | 1.08 | 1.08 | 1.08 | 1.08 |
| C16-18 fatty alcohol | 5 | 5 | 5 | 5 |
| Hydroxyethylcellulose | 0.2 | 0.2 | 0.2 | 0.2 |
| Methyl paraben | 0.2 | 0.2 | 0.2 | 0.2 |
| Formaldehyde | 0.1 | 0.1 | 0.1 | 0.1 |
| Silicone emulsion(4) | 5 | 0 | 2.5 | 2.5 |
| Dimethicone(DC200 fluid, 600,000 $mm^2s^{-1}$ (centistokes), from Dow Corning) | 0 | 2.95 | 0 | 0 |
| Amodimethicone emulsion(DC2-8177 emulsion from Dow Corning) | 0 | 0.05 | 0.05 | 0.05 |
| Silicone emulsion(1) | 0 | 0 | 0.45 | 0.45 |

(continued)

| Ingredient | Comp. Ex.E | Comp. Ex.F | Ex.17 | Ex.18 |
|---|---|---|---|---|
| PEG 150 distearate | 0 | 0.1 | 0.1 | 0.1 |
| Water | to 100 | to 100 | to 100 | to 100 |

The formulations were evaluated in a half head assessment, which was performed in salon by both hairdressers and panellists. The total number of panellists was 36, of which half had damaged hair and half normal hair.

Four tests were conducted.

In the first two tests a control conditioner formulation (Comparative Example E) was tested against Example 17 (test 1) and Comparative Example F (test 2) respectively.

Comparing the response of these two formulations relative to the control in these two tests demonstrates the benefit delivered by the silicone emulsion (1), since its omission in test 2 clearly reduced performance, despite the fact that the overall level of silicone active ingredient in the two formulations is the same.

In test 3 the formulations of Example 17 and Comparative Example F were compared directly. Example 17 demonstrated superior performance on wet and dry tactile attributes, when assessed by both hairdressers and panellists.

In test 4 Example 18 was compared against Comparative Example E. In this test, hairdresser assessment demonstrated clear superiority for Example 18 on wet tactile as well as styling benefits.

The results of Tests 1 to 4 were as follows:

Test 1

For damaged hair panellists, hairdresser assessment showed significant wins for Example 17 over Comparative Example E at 10% level or better for attributes of wet and dry softness, wet slip and dry smoothness, ease of dry combing, good alignment, ease of manageability and style retention.

Test 2

For damaged hair panellists no significant' differences were observed between Comparative Examples E and F.

Test 3

For all hair types, hairdresser assessment showed significant wins for Example 17 over Comparative Example F at 10% level or better for attributes of wet slip and wet softness, dry smoothness and wet and dry combing. Panellist assessment across all hair types showed significant wins for Example 17 at 10% level or better for attributes of wet slip and wet softness, dry softness, dry combing and ease of manageability.

Test 4

For all hair types, hairdresser assessment showed significant wins for Example 18 over Comparative Example E at 10% level or better for attributes of wet slip and wet softness, ease of wet combing, ease of manageability and good alignment.

## Claims

1. A hair shampoo or hair conditioner composition comprising a organosiloxane polymer having multiple branching points, in which the branching points are linked by organosiloxane segments having a chain length of at least 100 organosiloxy units, in which the branched organosiloxane polymer is **characterised by** the general formula:

$$Y[-C_nH_{2n}-(R_2SiO)_m-R_2Si-G]_x$$

where

Y is a tri- or tetravalent hydrocarbon radical,

R can be identical or different and is a monovalent hydrocarbon radical having from 1 to 18 carbon atoms per radical,
G is a monovalent radical of the formula $-C_fH_{2f-2k}-Z$ or a divalent radical of the formula $-C_nH_{2n}-$, the second bond being to a further radical Y,
Z is a monovalent hydrocarbon radical which is free from terminal aliphatic carbon-carbon multiple bonds, which is inert toward SiH groups in hydrosilylation reactions,
x is 3 or 4,
f is 2,
k is 0 or 1,
n is an integer.from 2 to 4, preferably 2,
m is an integer of at least 100 and with the proviso that the branched organosiloxane polymers contain on average at least one group z, wherein the composition is a hair shampoo comprising a cleansing anionic surfactant or a conditioner composition comprising a conditioning cationic surfactant.

**2.** A composition according to claim 1, in which m ranges from 100 to 300, more preferably from 200 to 300.

**3.** A composition according to claim 1 or 2, in which the branched organosiloxane polymer is **characterised by** the general formula:

$$C_6H_9[-C_2H_4-(Me_2SiO)_m-Me_2Si-G]_3$$

in which m ranges from 200 to 250, preferably about 220 to 240, G is a mixture of $-C_2H_4-$ and $-C_2H_4-C_6B_{13}$ groups and the group "$C_6H_9$" corresponds to a trivalent "1.2.4-cyclohexane triyl"-radical, and the molar ratio of $-C_2H_4-$ to $-C_2H_4-C_6H_{13}$ groups in the mixture G is about 2.1.

**4.** A composition according to any one of claims 1 to 3, which comprises one or more additional silicone emulsions formed from dimethicone, dimethiconol, amodimethicone or a mixture thereof.

**5.** A composition according to claim 4, which comprises an additional silicone emulsion formed from silicones and block copolymers of ethylene oxide and propylene oxide.

**6.** A process for preparing a composition according to any one of claims 1 to 5, in which the branched organosiloxane polymer is blended with a diluent, and the blend is mixed with the remaining composition ingredients.

**7.** A process according to claim 6, in which, the blend is emulsified prior to mixing with the remaining composition ingredients.

**8.** A process according to claim 6 or claim 7, in which the diluent is dimethicone fluid.

## Patentansprüche

**1.** Haarshampoo- oder Haarconditioner-Zusammensetzung, die ein Organosiloxan-Polymer mit Mehrfachverzweigungspunkten umfasst, in dem die Verzweigungspunkte durch Organosiloxan-Segmente mit einer Kettenlänge von wenigstens 100 Organosiloxy-Einheiten verknüpft sind, wobei das verzweigte Organosiloxan-Polymer durch die allgemeine Formel:

$$Y[-C_n-H_{2n}-(R_2SiO)_m-R_2Si-G]_x$$

gekennzeichnet ist, worin
Y ein drei- oder vierwertiger Kohlenwasserstoffrest ist,
R gleich oder unterschiedlich sein können und ein monovalenter Kohlenwasserstoffrest mit 1 bis 18 Kohlenstoffatomen pro Rest sind,
G ein monovalenter Rest der Formel $-C_fH_{2f-2k}-Z$ oder ein divalenter Rest der Formel $-C_nH_{2n}-$, wobei die zweite Bindung an einen weiteren Rest Y ist, ist,
Z ein monovalenter Kohlenwasserstoffrest ist, der frei von terminalen aliphatischen Kohlenstoff-Kohlenstoff-Mehrfachbindungen ist, der in Hydrosilylierungsreaktionen gegenüber SiH-Gruppen inert ist,
x 3 oder 4 ist,

f 2 ist,
k 0 oder 1 ist,
n eine ganze Zahl von 2 bis 4, vorzugsweise 2, ist,
n eine ganze Zahl von wenigstens 100 ist und
mit der Maßgabe, dass die verzweigten Organosiloxan-Polymere durchschnittlich wenigstens eine Gruppe Z enthalten, wobei die Zusammensetzung ein Haarshampoo ist, das ein anionisches Reinigungstensid umfasst, oder eine Conditioner-Zusammensetzung ist, die ein kationisches konditionierendes Tensid umfasst.

**2.** Zusammensetzung gemäß Anspruch 1, wobei m in Bereich von 100 bis 300, vorzugsweise von 200 bis 300, liegt.

**3.** Zusammensetzung gemäß Anspruch 1 oder 2, wobei das verzweigte Organosiloxan-Polymer durch die allgemeine Formel:

$$C_6H_9[-C_2H_4-(Me_2SiO)m-Me_2Si-G]_3$$

gekennzeichnet ist, in der m im Bereich vom 200 bis 250, vorzugsweise etwa 220 bis 240, liegt, G ein Gemisch aus Gruppen $-C_2H_4$ und $-C_2H_4-C_6H_{13}$ ist und die Gruppe "$C_6H_9$" einem trivalenten "1,2,4-Cyclohexantriyl"-Rest entspricht und das Molverhältnis der Gruppen $-C_2H_4-$ zu $-C_2H_4-C_6H_{13}$ in dem Gemisch G etwa 2:1 ist.

**4.** Zusammensetzung gemäß einem der Ansprüche 1 bis 3, die eine oder mehrere zusätzliche Silikonemulsionen umfasst, die aus Dimethicon, Dimethiconol, Amodimethicon oder einem Gemisch davon gebildet sind.

**5.** Zusammensetzung gemäß Anspruch 4, die eine zusätzliche Silikonemulsion umfasst, die aus Silikonen und Blockcopolymeren von Ethylenoxid und Propylenoxid gebildet ist.

**6.** Verfahren zur Herstellung einer Zusammensetzung gemäß einem der Ansprüche 1 bis 5, in dem das verzweigte Organosiloxan-Polymer mit einem Verdünnungsmittel gemischt wird und die Mischung mit den restlichen Zusammensetzungsingredienzien gemischt wird.

**7.** Verfahren gemäß Anspruch 6, wobei die Mischung vor Mischen mit dem verbleibenden Zusammensetzungsingredienzien emulgiert wird.

**8.** Verfahren gemäß Anspruch 6 oder Anspruch 7, wobei das Verdünnungsmittel ein Dimethicon-Fluid ist.

## Revendications

**1.** Composition de shampooing ou d'après-shampooing comprenant un polymère organosiloxane ayant de multiples points de ramification, dans laquelle les points de ramification sont reliés par des segments organosiloxane ayant une longueur de chaîne d'au moins 100 motifs organosiloxy, le polymère organosiloxane ramifié étant **caractérisé par** la formule générale :

$$Y[-C_nH_{2n}-(R_2SiO)_m-R_2Si-G]_x$$

où
Y est un radical hydrocarbure trivalent ou tétravalent,
les R peuvent être identiques ou différents et sont des radicaux hydrocarbure monovalents ayant de 1 à 18 atomes de carbone par radical,
G est un radical monovalent de formule $-C_fH_{2f-2k}-Z$ ou un radical divalent de formule $-C_nH_{2n}-$, la seconde liaison étant à un autre radical Y,
Z est un radical hydrocarbure monovalent qui est dépourvu des multiples liaisons carbone-carbone aliphatiques terminales, qui est inerte vis-à-vis des groupes SiH dans des réactions d'hydrosilylation,
x est 3 ou 4,
f est 2,
k est 0 ou 1,
n est un entier de 2 à 4, de préférence, 2,
m est un entier d'au moins 100 et
à condition que les polymères organosiloxane ramifiés contiennent en moyenne au moins un groupe Z, la

composition étant un shampooing comprenant un tensioactif de nettoyage anionique ou une composition d'après-shampooing comprenant un tensioactif de conditionnement cationique.

2. Composition selon la revendication 1, dans laquelle m va de 100 à 300, plus préférablement, de 200 à 300.

3. Composition selon les revendications 1 ou 2, dans laquelle le polymère organosiloxane ramifié est **caractérisé par** la formule générale :

$$C_6H_9[-C_2H_4-(Me_2SiO)_m-Me_2Si-G]_3$$

dans laquelle m va de 200 à 250, de préférence, d'environ 220 à 240, G est un mélange de groupes $-C_2H_4-$ et $-C_2H_4-C_6H_{13}$ et le groupe "$C_6H_9$" correspond à un radical "1.2.4-cyclohexane-triyle" trivalent, le rapport molaire des groupes $-C_2H_4-$ à $-C_2H_4-C_6H_{13}$ dans le mélange G étant d'environ 2:1.

4. Composition selon l'une quelconque des revendications 1 à 3, qui comprend une ou plusieurs émulsions de silicone supplémentaires formées à partir de diméthicone, diméthiconol, amodiméthicone ou un mélange de ceux-ci.

5. Composition selon la revendication 4, qui comprend une émulsion de silicone supplémentaire formée à partir de silicones et de copolymères séquencés d'oxyde d'éthylène et d'oxyde de propylène.

6. Procédé de préparation d'une composition selon l'une quelconque des revendications 1 à 5, dans lequel le polymère organosiloxane ramifié est homogénéisé avec un diluant, et le mélange homogénéisé est mélangé avec les ingrédients restants de la composition.

7. Procédé selon la revendication 6, dans lequel le mélange homogénéisé est émulsifié avant mélange avec les ingrédients restants de la composition.

8. Procédé selon la revendication 6 ou la revendication 7, dans lequel le diluant est un fluide de diméthicone.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20030055194 A
- WO 9206154 A
- US 5194639 A
- US 4009256 A
- WO 9522311 A
- US 3962418 A
- US 3958581 A
- WO 03094874 A